(19) Europäisches Patentamt
European Patent Office
Office européen des brevets

(11) **EP 4 389 007 A1**

(12) **EUROPEAN PATENT APPLICATION**

(43) Date of publication:
**26.06.2024 Bulletin 2024/26**

(21) Application number: **22290080.5**

(22) Date of filing: **19.12.2022**

(51) International Patent Classification (IPC):
**A61B 6/00** *(2024.01)*     **A61B 5/0285** *(2006.01)*
**G06T 7/00** *(2017.01)*      A61B 5/02 *(2006.01)*

(52) Cooperative Patent Classification (CPC):
**A61B 6/504; A61B 5/0285; A61B 6/461;**
**A61B 6/463; A61B 6/481; A61B 6/486;**
**A61B 6/507; G06T 7/0016;** A61B 5/02007;
G06T 2207/10016; G06T 2207/10076;
G06T 2207/10088; G06T 2207/10116;
G06T 2207/20084; G06T 2207/30048;     (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **Florent, Raoul**
**5656 AG Eindhoven (NL)**

• **This, Alexandre**
**5656 AG Eindhoven (NL)**
• **Schmitt, Holger**
**5656 AG Eindhoven (NL)**
• **Levrier, Claire**
**5656 AG Eindhoven (NL)**
• **Van Der Horst, Arjen**
**5656 AG Eindhoven (NL)**

(74) Representative: **Philips Intellectual Property &**
**Standards**
**High Tech Campus 52**
**5656 AG Eindhoven (NL)**

(54) **EVALUATION OF BLOOD FLOW PARAMETERS**

(57)     A system (100) for evaluating blood flow parameters includes one or more processors (110). The processor(s) analyse angiographic image data (120) representing a motion of a front ($130_1$, $130_1$) of an injected contrast agent along a vessel (140) in each of a first haemodynamic state and a second haemodynamic state, to determine a transit time, or a transit velocity, for the vessel (140) in each state. A value of one or more blood flow parameters (160) for the vessel (140) is output. The value of the blood flow parameter(s) is calculated based on the values of the transit times or the values of the transit velocities. A first angiogram ($170_1$) and a second angiogram ($170_2$), the first angiogram and the second angiogram are outputted. The angiograms depict the motion of the fronts ($130_1$, $130_2$) along the vessel (140) in the first haemodynamic state and the second haemodynamic state, respectively. The motion of the fronts ($130_1$, $130_2$) depicted in the first angiogram ($170_1$) and the second angiogram ($170_2$), is synchronised such that the fronts in both angiograms . simultaneously leave the proximal position ($150_p$) in the vessel.

FIG. 5

**(Cont. next page)**

EP 4 389 007 A1

(52) Cooperative Patent Classification (CPC): (Cont.)
   G06T 2207/30104

**Description**

**Technical Field**

The present disclosure relates to the evaluation of blood flow parameters. A system, a computer-implemented method, and a computer program product, are disclosed.

**Background**

Various parameters have been developed for assessing blood flow in the vasculature. Some of these parameters are evaluated using measurements of the blood flow in two different haemodynamic states. For instance, the Coronary Flow Reserve "CFR" is a blood flow parameter that is used as a measure of the ability of the coronary arteries to respond to an increase in the heart's demand for oxygen. The CFR is calculated from measurements of the blood flow in both the basal state, i.e. the resting state, and also in the hyperemic state, i.e. when blood flow is increased as compared to the basal state. The Maximal Flow Ratio "MFR" is another example of a blood flow parameter that may be evaluated using measurements of the blood flow in two different haemodynamic states. The MFR is a measure of the change in maximal blood flow as a result of an intravascular procedure, such as percutaneous transluminal coronary angioplasty, "PCTA". The MFR is calculated from measurements of the blood flow in both a pre-procedural haemodynamic state, and also in a post-procedural haemodynamic state in order to compare the blood flow between the two states. Other blood flow parameters, including the Index of Microcirculatory Resistance "IMR" may be evaluated separately for each of two different haemodynamic states using measurements of the blood flow from only the corresponding state. For instance, a value for the IMR may be calculated from measurements of the blood flow in each of a pre-procedural haemodynamic state, and a post-procedural haemodynamic state, in order to compare the blood flow between the two states.

The techniques that are currently available for measuring blood flow parameters include invasive techniques, and angiographic techniques. Invasive techniques involve the use of intravascular temperature-sensing devices, flow-sensing devices, and pressure-sensing devices, to measure blood flow. Angiographic techniques involve the injection of a contrast agent into the vasculature in order to provide visibility of the blood flow with imaging techniques such as X-ray, and magnetic resonance imaging "MRI". Features in the resulting images are then analysed in order to evaluate the blood flow. One group of such angiographic imaging techniques involves the measurement of blood flow by tracking a front of an injected contrast agent bolus and determining the so-called "transit time" or the "transit velocity" in a blood vessel. The transit time is defined as the time taken for the front of the injected contrast agent to pass between specified proximal and distal positions in a vessel. The transit velocity is defined as the average speed of the front of the injected contrast agent between specified proximal and distal positions in a vessel.

Angiographic techniques for measuring blood flow parameters are often preferred over invasive techniques due to their reduced procedural complexity. However, physicians that are more familiar with invasive techniques can be suspicious of the reliability of blood flow parameters that have been measured angiographically. This hampers the adoption of angiographic techniques for measuring blood flow parameters, and presents a barrier to the exploitation of their benefits. Consequently there is a need to provide improved confidence in the values of angiographically-derived measurements of blood flow parameters.

**Summary**

According to one aspect of the present disclosure, a system for evaluating blood flow parameters, is provided. The system includes one or more processors configured to:

- receive angiographic image data representing a motion of a front of an injected contrast agent along a vessel in each of a first haemodynamic state and a second haemodynamic state;
- analyse the angiographic image data to determine a transit time or a transit velocity, for the vessel in each state, the transit time in each state being calculated based on a time taken for the front to pass between a proximal position in the vessel and a distal position in the vessel, and the transit velocity in each state being calculated based on one or more distances traversed by the front between the proximal position in the vessel and the distal position in the vessel, and one or more corresponding time intervals;
- output a value of one or more blood flow parameters for the vessel, the value of the one or more blood flow parameters being calculated based on the values of the transit times, or the values of the transit velocities; and
- output a first angiogram and a second angiogram, the first angiogram and the second angiogram being generated from the angiographic image data and depicting the motion of the fronts along the vessel in the first haemodynamic state and the second haemodynamic state, respectively; and

- wherein the motion of the fronts depicted in the first angiogram, and the second angiogram, is synchronised such that the fronts in both angiograms simultaneously leave the proximal position in the vessel.

The system outputs a value of one or more blood flow parameters for a vessel. The one or more blood flow parameters are evaluated based on the values of transit times, or transit velocities, and which are calculated in two different haemodynamic states. The haemodynamic states may be the basal state, and the hyperemic state, or a pre-procedural haemodynamic state, and a post-procedural haemodynamic state, for instance. Since the transit times, or the transit velocities, are calculated using measurements that are made from angiographic image data, the blood flow parameter(s) are provided with reduced procedural complexity as compared to calculating the transit times, or the transit velocities, using measurements that are made using invasive techniques. The system also outputs a first angiogram, and a second angiogram. The angiograms depict the motion of the fronts along the vessel in the first haemodynamic state, and the second haemodynamic state, respectively. The motion of the fronts depicted in the angiograms is synchronised such that the fronts in both angiograms simultaneously leave the proximal position in the vessel. The angiograms enable a physician to observe the relative motion of the fronts along the vessel. Consequently, the physician may observe from the relative motion of the fronts along the vessel that the measurements of the blood flow that underly the calculated value of the blood flow parameter(s) are physiologically sound. Thus, the angiograms provide insights into the angiographic measurement of the transit times, or transit velocities, that underly the calculated value of the blood flow parameter(s), and these insights help to improve a physician's confidence in the calculated value(s).

Further aspects, features, and advantages of the present disclosure will become apparent from the following description of examples, which is made with reference to the accompanying drawings.

**Brief Description of the Drawings**

Fig. 1 is a schematic diagram illustrating an example of a system 100 for evaluating blood flow parameters, in accordance with some aspects of the present disclosure.
Fig. 2 is a flowchart illustrating an example of a method of evaluating blood flow parameters, in accordance with some aspects of the present disclosure.
Fig. 3 is a schematic diagram illustrating an example of a vessel 140 in the heart, including an example of a proximal position $150_p$ and a distal position $150_d$ in the vessel 140, in accordance with some aspects of the present disclosure.
Fig. 4 is a graph illustrating an example of the distance, Length, 190, traversed by a front from a reference position in the vessel at the times, Frame #, of corresponding angiographic images, in accordance with some aspects of the present disclosure.
Fig. 5 is an example of a first angiogram $170_1$ and a second angiogram $170_2$ depicting the motion of a front $130_1$, $130_2$ of an injected contrast agent along a vessel 140 in a first haemodynamic state, and a second haemodynamic state, respectively, in accordance with some aspects of the present disclosure.
Fig. 6 is a second example of a first angiogram $170_1$ and a second angiogram $170_2$ depicting the motion of a front $130_1$, $130_2$ of an injected contrast agent along a vessel 140 in a first haemodynamic state, and a second haemodynamic state, respectively, in accordance with some aspects of the present disclosure.

**Detailed Description**

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer implemented method, and in a computer program product, in a corresponding manner.

In the following description, reference is made to a system for evaluating blood flow parameters. In some examples, blood flow parameters are evaluated for a coronary vessel. For example, reference is made to examples in which blood flow parameters are evaluated for a coronary artery. However, it is to be appreciated that this serves only as an example. In general, the system may be used to evaluate blood flow parameters in any blood vessel in the vasculature. Thus, the vessel may for instance be an artery, or a vein, or a network thereof. Moreover, the vessel may be in any region in the anatomy. Thus, the vessel may be disposed in a region of the body such as the heart, the brain, the leg, the lung, and so forth.

In the following description, reference is made to examples in which values of blood flow parameters are calculated based on measurements of the transit time, or the transit velocity, in the vessel, in two haemodynamic states. In some examples, the states are the basal state, and the hyperemic state. However, it is to be appreciated that these states are only provided as examples. In general, the system may be used to calculate the values of blood flow parameters for any two haemodynamic states. For instance, the values of blood flow parameters may be calculated based on measurements of the transit time, or the transit velocity, in a pre-procedural haemodynamic state, and in a post-procedural haemodynamic state.

In the following description, reference is made to examples in which the blood flow parameter that is evaluated by the system is the CFR. The CFR is an example of a blood flow parameter that is evaluated using measurements of the blood flow in two different states. However, the system is not limited to use in evaluating this specific blood flow parameter. In general, the system may be used to evaluate any blood flow parameter that can be evaluated using measurements of a transit time, or a transit velocity, in a vessel in two different haemodynamic states. For instance, the system may alternatively be used to evaluate blood flow parameters such as the MFR and the IMR. It is noted that the CFR, and also the MFR, are evaluated based on the measurements of a transit time, or a transit velocity, in a vessel in both of two different haemodynamic states. However, the IMR may be evaluated separately for each of two different haemodynamic states using measurements of the blood flow from only the corresponding state. The IMR, and also other blood flow parameters, are often evaluated separately for each of two different haemodynamic states in order to in order to compare blood flow between the two states. In the example of the IMR, the IMR may be evaluated separately for a pre-procedural haemodynamic state, and a post-procedural haemodynamic state. Thus, in general, the system may be used to evaluate a single blood flow parameter using measurements of a transit time, or a transit velocity, in a vessel in both of two different haemodynamic states, or alternatively, the system may be used to evaluate a blood flow parameter separately for each of two different haemodynamic states wherein the blood flow parameter is evaluated for each haemodynamic state using measurements of the blood flow from only the corresponding state. In the latter case, the separate values of the blood flow parameter for each of the two states may also be combined into a single blood flow parameter. For example, a ratio may be evaluated using their individual values in order to express the relative change between the two states via a single parameter.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, the Internet, or the Cloud. The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

As mentioned above, various parameters have been developed for assessing blood flow in the vasculature. Some of these parameters may be evaluated using measurements of the blood flow in two different haemodynamic states.

The Coronary Flow Reserve "CFR" is an example of one such blood flow parameter. The CFR is a measure of the ability of the coronary arteries to respond to an increase in the heart's demand for oxygen. The CFR is calculated from measurements of the blood flow in both the basal state, and also in the hyperemic state. The Coronary Flow Reserve "CFR" is defined as the ratio of coronary blood flow in the hyperemic state to the coronary blood flow in the basal state. When the demand for oxygen in the myocardium is increased, the vascular resistance of the coronary arteries and the related vasculature bed have the ability to reduce. In-turn, this reduction in vascular resistance leads to an increase in the blood flow to the myocardium. During this process, all sections along the path of the related vasculature, i.e. the artery, the arterioles, and also the capillaries, can respond by changing their lumen diameter. A malfunctioning at any section along the path will be reflected by a decrease in the value of the CFR. A low value of the CFR can therefore be indicative of epicardial (artery) or myocardial (arterioles and capillaries) problems. The former is referred-to as CAD "Coronary Artery Disease" and the second is referred-to as NOCAD "Non Obstructive Coronary Artery Disease". Values of the CFR that are below a threshold of approximately 2.0, are typically diagnosed as a vascular malfunction. If there is no diagnosed epicardial stenosis, this often leads to a diagnosis of Micro Vascular Disease "MVD".

The Maximal Flow Ratio "MFR" is another example of a blood flow parameter that may be evaluated using measurements of the blood flow in two different haemodynamic states. The MFR is a measure of the change in maximal blood flow as a result of an intravascular procedure, such as percutaneous transluminal coronary angioplasty, "PCTA". The MFR is calculated from measurements of the blood flow in both a pre-procedural haemodynamic state, and also in a post-procedural haemodynamic state in order to compare the blood flow between the two states. The MFR is measured in each of these states during maximal hyperemia, i.e. with the vessel in the hyperemic state. The MFR may be used to provide immediate information about the result of the procedure, and is described further in a document by Pijls, N. H., et al., "Concept of maximal flow ratio for immediate evaluation of percutaneous transluminal coronary angioplasty result by videodensitometry", Circulation. 1991; 83:854-865.

The Index of Microcirculatory Resistance "IMR" is an example of a blood flow parameter that may be evaluated separately for each of two different haemodynamic states using measurements of the blood flow from only the corresponding state. The IMR is used to assess coronary microvascular function, and provides information on microvascular dysfunction that could be informative both in patients with stable coronary arterial disease, and also in patients with acute or recent myocardial infarction. The IMR may be calculated from measurements of the blood flow in a pre-procedural haemodynamic state, and a post-procedural haemodynamic state, using measurements of the blood flow from only the corresponding state. The IMR is evaluated separately in each state in order to compare blood flow between the two states. Measurements of the blood flow in each state that are used to calculate the IMR are typically performed at maximal hyperemia. The IMR values for the two states may also be combined into a single blood flow parameter. For example, a ratio may be evaluated using their individual values in order to express the relative change between the two states via a single parameter.

Other parameters for assessing blood flow may also be evaluated using measurements of the blood flow in two different haemodynamic states.

The techniques that are currently available for measuring blood flow parameters include invasive techniques, and angiographic techniques. Invasive techniques involve the use of intravascular temperature-sensing devices, flow-sensing devices, and pressure-sensing devices, to measure blood flow. Angiographic techniques involve the injection of a contrast agent into the vasculature in order to provide visibility of the blood flow with imaging techniques such as X-ray, and magnetic resonance imaging "MRI". Features in the resulting images are then analysed in order to evaluate the blood flow. One group of such angiographic imaging techniques involves the measurement of blood flow by tracking a front of an injected contrast agent bolus and determining the so-called "transit time" or the "transit velocity" in a blood vessel. The transit time is defined as the time taken for the front of the injected contrast agent to pass between specified proximal and distal positions in a vessel. The transit velocity is defined as the average speed of the front of the injected contrast agent between specified proximal and distal positions in a vessel.

With reference to the example blood flow parameters described above, the value of the CFR can be evaluated using angiographic techniques by tracking the front of the injected contrast agent as it travels between the proximal position and the distal position in a vessel. Assuming that the same proximal and distal positions are chosen in each of the hyperaemic and basal states, and assuming that the diameter of the vessel is only marginally impacted by the change in state, then the CFR may be evaluated using Equation 1:

$$CFR = \frac{Q_h}{Q_b} \approx \frac{A\,V_h}{A\,V_b} = \frac{V_h}{V_b} = \frac{L}{TT_h}\frac{TT_b}{L} = \frac{TT_b}{TT_h} \qquad \text{Equation 1}$$

In this equation, the subscripts h and *b* refer to the hyperaemic and basal states respectively, Q refers to blood flow rate, A refers to average vessel cross section, V refers to the blood velocity, L refers to the vessel's length between the proximal and distal positions, and $TT_b$ and $TT_h$ refer to the transit-time over length L in the basal state *b,* and in the hyperemic state *h*, respectively.

The evaluation of the CFR therefore amounts to a calculation of a ratio of the transit times in the basal and hyperemic states. The transit time is measured in the basal state under resting conditions. The transit time is often measured in the hyperemic state by triggering the hyperemic state using an intravenous or intraarterial injection of a vasodilation drug such as Adenosine. The value of the CFR may be evaluated for any coronary artery. For example, it may be calculated for the left anterior descending "LAD" artery, or the left circumflex coronary artery "LCx". As indicated in Equation 1, the CFR may alternatively be evaluated with Equation 1 using the transit velocity in the basal state, $V_b$, and the transit velocity in the hyperemic state, $V_h$. The transit velocity is defined as the average speed of the front of the injected contrast agent between specified proximal and distal positions in a vessel.

The MFR is another example of a blood flow parameter that may be evaluated using angiographic techniques. The MFR is calculated from measurements of the blood flow in both a pre-procedural haemodynamic state, and also in a post-procedural haemodynamic state in order to compare the blood flow between the two states. The MFR is measured in each of these states during maximal hyperemia, i.e. with the vessel in the hyperemic state. The value of the MFR may be determined by measuring a transit time taken for an injected bolus to travel between a proximal position in a vessel, and a distal position in the vessel. The MFR may be evaluated using Equation 2:

$$MFR = \frac{TT_{Pre}}{TT_{Post}} \qquad\qquad \text{Equation 2}$$

In Equation 2, $TT_{Pre}$ refers to the transit time in the pre-procedural haemodynamic state, and $TT_{Post}$ refers to the transit time in the post-procedural haemodynamic state. Instead of using the transit time $TT$ in Equation 2, the transit times may be substituted for the ratio of the distance between the proximal position and the distal position, L, to the transit velocity, $V$, between these positions, i.e. $L/V$, in the corresponding state.

The IMR is another example of a blood flow parameter that may be evaluated using angiographic techniques. A value for the IMR may be calculated for each of two different states, such as a pre-procedural haemodynamic state, and a post-procedural haemodynamic state, in order to compare blood flow between the two states. The IMR values for the two states may also be combined into a single blood flow parameter, for example by evaluating a ratio from their individual values. The value of the IMR may be determined by measuring a transit time taken for an injected bolus to travel between a proximal position in a vessel, and a distal position in the vessel. The measurements of the transit time in each state are typically performed at maximal hyperemia. The IMR may be evaluated using Equation 3:

$$IMR = P_d \cdot TT \qquad\qquad \text{Equation 3}$$

In Equation 3, $P_d$ represents the distal pressure at the distal position in the vessel, and $TT$ represents the transit time. The IMR is typically calculated with Equation 3 using the time-averaged value of the distal pressure $P_d$ over a cardiac cycle at maximal hyperemia. Instead of using the transit time $TT$ in Equation 3, the transit time may be substituted for the ratio of the distance between the proximal position and the distal position, L, to the transit velocity, $V$, between these positions, i.e. $L/V$, in the corresponding state.

A value for the distal pressure $P_d$, in Equation 3 may be obtained angiographically, or using invasive techniques. In the former case, the distal pressure $P_d$ may be estimated using an invasive measurement of the proximal pressure $P_a$ and a linear estimation of the pressure drop between $P_a$ and $P_d$, that is based on angiographically-derived vessel measurements. The pressure drop may be estimated using the Hagen-Poiseuille equation, for example.. The vessel measurements may be obtained by segmenting one or more angiographic images of the vessel. Various image segmentation techniques are known for this purpose, including thresholding, template matching, active contour modelling, model-based segmentation, neural networks, e.g., U-Nets, and so forth.

Alternative equations to Equation 3 may also be used to compute the IMR, and these likewise depend on the transit time $TT$, or correspondingly, the transit velocity, $V$. For instance, if a vessel is subject to severe epicardial stenosis, an alternative definition for the IMR has been proposed, and wherein the IMR is computed using Equation 4:

$$IMR = P_d \cdot TT \cdot \left(\frac{P_d - P_w}{P_a - P_w}\right) \qquad\qquad \text{Equation 4}$$

As compared to Equation 3, in Equation 4, the additional term $P_a$ represents the proximal pressure at the proximal position in the vessel, and the additional term $P_w$ represents the coronary wedge pressure, i.e. the pressure at a distal position to the stenosis when the vessel is occluded by an inflated balloon. The IMR is typically calculated with Equation 4 using the time-averaged value of the proximal pressure $P_a$ over a cardiac cycle at maximal hyperemia, using the time-averaged value of the distal pressure $P_d$ over a cardiac cycle at maximal hyperemia, and using the time-averaged value of the pressure over a cardiac cycle at the distal position $Pos_d$ in the vessel when the vessel is occluded by an inflated balloon the wedge pressure $P_w$. A value for the wedge pressure $P_w$ in Equation 4 may be measured in a similar manner to that described above for the distal pressure $P_d$. Thus, the value of the wedge pressure $P_w$ may be measured using an intraluminal pressure sensor. Alternatively, the wedge pressure $P_w$ may be estimated using a geometric model in a similar manner to the estimated distal pressure $P_d$.

Angiographic techniques for measuring blood flow parameters are often preferred over invasive techniques due to their reduced procedural complexity. However, physicians that are more familiar with invasive techniques can be suspicious of the reliability of blood flow parameters that have been measured angiographically. This hampers the adoption of angiographic techniques for measuring blood flow parameters, and presents a barrier to the exploitation of their benefits. Consequently there is a need to provide improved confidence in the values of angiographically-derived measurements of blood flow parameters.

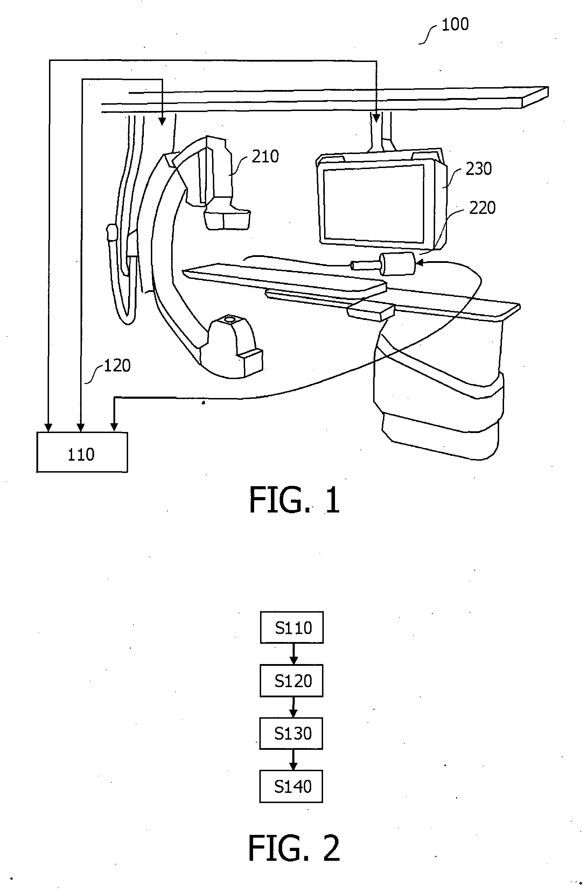

Fig. 1 is a schematic diagram illustrating an example of a system 100 for evaluating blood flow parameters, in accordance with some aspects of the present disclosure. The system 100 includes one or more processors 110. Fig. 2 is a flowchart illustrating an example of a method of evaluating blood flow parameters, in accordance with some aspects of the present disclosure. It is noted that the operations described in relation to the one or more processors 110 of the system 100 illustrated in Fig. 1, may also be performed in the method illustrated in Fig. 2. Likewise, operations described in relation to the method illustrated in Fig. 2, may also be performed by the one or more processors 110 of the system 100 illustrated in Fig. 1.

With reference to Fig. 1, the one or more processors 110 are configured to:

- receive S110 angiographic image data 120 representing a motion of a front $130_1$, $130_2$ of an injected contrast agent along a vessel 140 in each of a first haemodynamic state and a second haemodynamic state;
- analyse S120 the angiographic image data 120 to determine a transit time, or a transit velocity, for the vessel 140 in each state, the transit time in each state being calculated based on a time taken for the front to pass between a proximal position $150_p$ in the vessel and a distal position $150_d$ in the vessel, and the transit velocity in each state being calculated based on one or more distances traversed by the front between the proximal position $150_p$ in the vessel and the distal position $150_d$ in the vessel, and one or more corresponding time intervals;

- output S130 a value of one or more blood flow parameters 160 for the vessel 140, the value of the one or more blood flow parameters being calculated based on the values of the transit times or the values of the transit velocities; and
- output S140 a first angiogram $170_1$ and a second angiogram $170_2$, the first angiogram and the second angiogram being generated from the angiographic image data 120 and depicting the motion of the fronts $130_1$, $130_2$ along the vessel 140 in the first haemodynamic state and the second haemodynamic state, respectively; and
- wherein the motion of the fronts $130_1$, $130_2$ depicted in the first angiogram $170_1$, and the second angiogram $170_2$, is synchronised such that the fronts in both angiograms simultaneously leave the proximal position $150_p$ in the vessel.

The system outputs a value of one or more blood flow parameters for a vessel. The one or more blood flow parameters are evaluated based on the values of transit times, or transit velocities, and which are calculated in two different haemodynamic states. The haemodynamic states may be the basal state, and the hyperemic state, or a pre-procedural haemodynamic state, and a post-procedural haemodynamic state, for instance. Since the transit times, or the transit velocities, are calculated using measurements that are made from angiographic image data, the blood flow parameter(s) are provided with reduced procedural complexity as compared to calculating the transit times, or the transit velocities, using measurements that are made using invasive techniques. The system also outputs a first angiogram, and a second angiogram. The angiograms depict the motion of the fronts along the vessel in the first haemodynamic state, and the second haemodynamic state, respectively. The motion of the fronts depicted in the angiograms is synchronised such that the fronts in both angiograms simultaneously leave the proximal position in the vessel. The angiograms enable a physician to observe the relative motion of the fronts along the vessel. Consequently, the physician may observe from the relative motion of the fronts along the vessel that the measurements of the blood flow that underly the calculated value of the blood flow parameter(s) are physiologically sound. Thus, the angiograms provide insights into the angiographic measurement of the transit times, or transit velocities, that underly the calculated value of the blood flow parameter(s), and these insights help to improve a physician's confidence in the calculated value(s).

With reference to Fig. 1, and Fig. 2, in the operation S110, the one or more processors 110 receive angiographic image data representing a motion of a front $130_1$, $130_2$ of an injected contrast agent along a vessel 140 in each of a first haemodynamic state, and a second haemodynamic state.

In general, the angiographic image data 120 that is received in the operation S110 may be 2D image data, or alternatively it may be volumetric image data. The angiographic image data may be generated by an X-ray imaging system, or by an MRI imaging system. In the former case, the X-ray imaging system may be a projection X-ray imaging system, or it may be a volumetric X-ray imaging system. In this case, the angiographic image data 120 may be projection X-ray image data, or it may be volumetric X-ray image data, as described in the examples below. In the latter case, an MRI imaging system typically generates volumetric image data, and consequently the angiographic image data 120 may be volumetric MRI image data. The volumetric MRI image data generated by an MRI imaging system may also be projected from a defined orientation using ray tracing techniques to provide projection MRI image data.

Projection X-ray imaging systems often include a common support arm such as a so-called "C-arm", or an "O-arm", that supports an X-ray source-detector arrangement. Projection X-ray imaging systems may alternatively include a support arm with a different shape to these examples. In some projection X-ray imaging systems, the X-ray source and X-ray detector are supported separately, and there is consequently no common support arm. One example of a projection X-ray imaging system is a biplane projection X-ray imaging system, and which employs two pairs of X-ray source-detector arrangements. A projection X-ray imaging system may be used to generate angiographic image data from two different orientations with respect to a region of interest. The orientations are typically arranged in a transverse manner, and in some examples the orientations are mutually orthogonal. Projection X-ray imaging systems typically generate image data with the X-ray source and X-ray detector held in a static position with respect to an imaging region. The image data generated by a projection X-ray imaging system may be referred-to as projection X-ray image data. An example of a projection X-ray imaging system is the Philips Azurion 7 X-ray imaging system marketed by Philips Healthcare, Best, The Netherlands.

Volumetric X-ray imaging systems, by contrast, typically generate image data whilst rotating, or stepping, an X-ray source-detector arrangement around an imaging region. The image data that is generated from multiple rotational angles with respect to the imaging region may subsequently be reconstructed into a volumetric image. The image data generated by a volumetric X-ray imaging system may be referred-to as volumetric X-ray image data, or CT image data. Examples of volumetric X-ray imaging systems include computed tomography "CT" imaging systems, cone beam CT "CBCT" imaging systems, and spectral CT imaging systems. An example of a CT imaging system is the CT 5000 Ingenuity CT scanner that is marketed by Philips Healthcare, Best, The Netherlands.

Projection X-ray imaging systems may also generate image data whilst rotating, or stepping, an X-ray source-detector arrangement around an imaging region. Image reconstruction techniques may then be used to reconstruct the projection X-ray image data that is obtained from multiple rotational angles with respect to the object, into a volumetric image, in a similar manner to the reconstruction of a volumetric image using volumetric X-ray image data that is generated by a CT imaging system. Volumetric X-ray image data may therefore be generated by an X-ray projection imaging system.

The angiographic image data 120 that is received in the operation S110 represents a motion of a front $130_1$, $130_2$ of an injected contrast agent along a vessel 140 in each of a first haemodynamic state, and a second haemodynamic state. The first and second haemodynamic states may be the basal state, and the hyperemic state, respectively. The first and second haemodynamic states may alternatively be any two haemodynamic states, such as a pre-procedural state, and a post-procedural state, for example. A vessel in the basal, i.e. resting, state refers to the blood flow when the body is operating at the basal metabolic rate, i.e. when the body is using the amount of energy per unit of time that a person needs to keep the body functioning at rest. A vessel in the hyperemic state refers to the blood flow being increased as compared to the basal state. As mentioned above, the hyperemic state may be induced by intravenous or intraarterial injection of a vasodilation drug such as Adenosine.

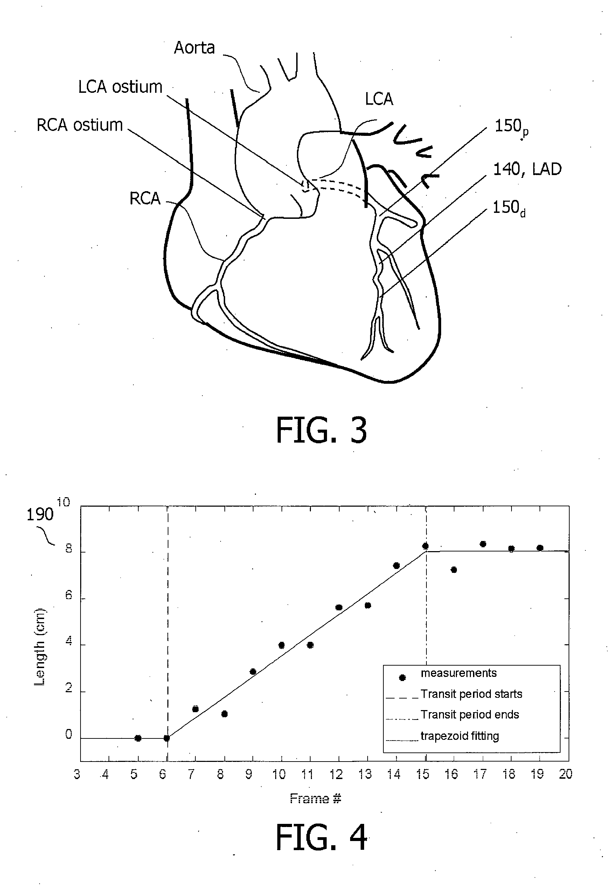

The angiographic image data 120 that is received in the operation S 120 represents a motion of a front $130_1$, $130_2$ of an injected contrast agent along a vessel 140. Thus, the angiographic image data 120 may include a temporal sequence of images. In examples in which the data is X-ray image data, such images may be referred-to as "fluoroscopic", or "cine-angiographic", images. The temporal sequence of images captures the front of the injected contrast agent as the front passes along the vessel 140. In general, the vessel may be located anywhere in the body, such as in the heart, the brain, the leg, the lung, and so forth. By way of an example, Fig. 3 is a schematic diagram illustrating an example of a vessel 140 in the heart, including an example of a proximal position $150_p$ and a distal position $150_d$ in the vessel 140, in accordance with some aspects of the present disclosure. The vessel 140 illustrated in Fig. 3 is the left anterior descending artery, LAD. Thus, the angiographic image data 120 that is received in the operation S120 may represent a motion of a front $130_1$, $130_2$ of an injected contrast agent along a vessel such as the LAD in the heart illustrated in Fig. 3, for example.

The angiographic image data 120 that is received in the operation S120 represents a motion of a front $130_1$, $130_2$ of an injected contrast agent along a vessel 140. The contrast agent may be injected into the body in the form of a so-called "bolus". The contrast agent may be injected directly into the vessel, or it may be injected at another position in the vasculature of a subject, and from which position the contrast agent flows into the vessel. The contrast agent may include a substance such as Iodine, or a Lanthanide such as Gadolinium, or indeed another substance that provides visibility of blood flow under angiographic imaging. The contrast agent may be injected using a syringe. The syringe may be operated via a contrast agent injector such as the injector 220 illustrated in Fig. 1. The injector 220 may be controlled manually, or automatically. In the latter case, the one or more processors 110 may issue a trigger signal to trigger the injection of the contrast agent. An example of a suitable injector is the Medrad Mark 7 Arterion ™ Injection System marketed by Medrad Europe, B.V., Beek, The Netherlands. This, however, is mentioned purely by way of an example.

The received angiographic image data may include a temporal sequence of images representing a motion of a front of an injected contrast agent along a vessel in each of the first haemodynamic state, and the second haemodynamic state. Thus, the angiographic image data 120 that is received in the operation S120 may represent an angiogram that depicts the motion of a front of an injected contrast agent along a vessel in a first haemodynamic state, and an angiogram that depicts the motion of a front of an injected contrast agent along the vessel in a second haemodynamic state.

Referring back to Fig. 2, in the operation S120, the one or more processors of the system 100 analyse S120 the angiographic image data 120 to determine a transit time, or a transit velocity, for the vessel 140 in each state. The transit time in each state is calculated based on a time taken for the front to pass between a proximal position $150_p$ in the vessel, and a distal position $150_d$ in the vessel, and the transit velocity in each state is calculated based on one or more distances traversed by the front between the proximal position $150_p$ in the vessel, and the distal position $150_d$ in the vessel, and one or more corresponding time intervals.

Various techniques are contemplated for determining the transit times, or the transit velocities, in the operation S 120, some of which are described in the examples below. In general, the transit times, or transit velocities, are calculated using a proximal position $150_p$ in the vessel, and a distal position $150_d$. The terms proximal position $150_p$ and distal position $150_d$ used herein are defined with respect to the ostium of the relevant vessel. The proximal position and distal positions also refer to positions with respect to conventional blood flow in the vessel; the proximal position being relatively upstream with respect to conventional blood flow in the vessel, and the distal position being relatively downstream with respect to conventional blood flow in the vessel. Thus, with reference to the example vessel illustrated in Fig. 3, the proximal position $150_p$ is proximal to the LAD ostium, and the distal position $150_d$ is distal to the LAD ostium. In the determination of some blood flow parameters, the proximal position $150_p$ may correspond to an anatomical landmark. For example, the LAD ostium, or a bifurcation in the vessel may be chosen as the proximal position. In the determination of some blood flow parameters, the proximal position $150_p$ may correspond to an interventional device landmark. For example, if an injection catheter is visible in the angiographic image data, a position on the injection catheter may be chosen as the proximal position. In the determination of some blood flow parameters, the distal position $150_d$ may refer more specifically to a position in the "distal two-thirds" of the target lumen, as mentioned in a document by Kobayashi, Y., and Fearon, W. F., "Invasive coronary microcirculation assessment - Current status of Index of Microcirculatory Resistance", Circulation Journal, Official Journal of the Japanese Circulation Society, 2014; 78(5); pages 1021-1028. In the determination of some blood flow parameters, the distal position $150_d$ may refer to the most distal position in the vessel in an angiographic image.

An example of the operation S120 in which the angiographic image data is analysed to determine a transit time for the vessel is now described with reference to Fig. 3 and Fig. 4 for the example vessel 140 illustrated in Fig. 3. In this example, the received angiographic image data 120 comprises a temporal sequence of images representing the motion of the front $130_1$, $130_2$ of the injected contrast agent along the vessel 140 in each of the first haemodynamic state, and the second haemodynamic state. The one or more processors 110 are configured to generate the first angiogram $170_1$, and the second angiogram $170_2$, from the temporal sequence of images representing the first haemodynamic state, and the second haemodynamic state, respectively. The one or more processors 110 are also configured to:

- analyse the angiographic image data 120 to determine the transit time, or the transit velocity, for the vessel 140 in each of the first haemodynamic state, and the second haemodynamic state, by:
- tracking a position of the front $130_1$, $130_2$ in the temporal sequence of images;
- defining the proximal position $150_p$ and the distal position $150_d$ in the vessel 140; and
- calculating the transit time, or the transit velocity, based on the tracked positions of the front in relation to the proximal position $150_p$ and the distal position $150_d$.

Various examples of the operation of tracking a position of the front in the images in the temporal sequence are described in the examples below.

In one approach, a target vessel is tracked in the temporal sequence of images, and the position of the contrast agent front is identified in the target vessel. In this approach, a classification technique, such as neural network or a feature detector, is trained to identify a target vessel in the images in the temporal sequence. For instance, a neural network may be trained to identify a target vessel in an angiographic image, following which, the trained neural network is used to identify the target vessel in the images in the temporal sequence. The neural network may be a convolutional neural network "CNN", and the target vessel may be the LAD illustrated in Fig. 3, for example. In this approach, having identified the target vessel in a given image in the temporal sequence of images, the position of the contrast agent front is then identified in the image by measuring a length of the vessel from a reference position in the vessel, and defining the position of the contrast agent front as the furthest point in the vessel from the reference position. The reference position may be chosen at a position that is close to the ostium of the vessel, for example. In this approach, prior to using such classification techniques to track the target vessel, the images in the temporal sequence may be segmented in order to identify the path of the contrast agent. This improves the reliability of the classification.

In another approach, instead of tracking a target vessel in the temporal sequence of images, a surrogate vessel is tracked, and the position of the contrast agent front is identified in the surrogate vessel. The surrogate vessel may be defined as the longest vessel in a given image in the temporal sequence, for example. Since this approach is based on a determination of the longest vessel in a given image, it inherently identifies the position of the contrast agent front in the images. In this approach, prior to tracking the surrogate vessel, the images in the temporal sequence may be segmented in order to identify the path of the contrast agent. This improves the reliability of the tracking of the surrogate vessel.

In this approach, the longest vessel in a given image may be identified by determining the longest of the shortest paths in an image between a reference position and any other position in the vasculature. The reference position may be chosen at a position that is close to the ostium of a vessel, for example. A shortest path between the reference position and any position in the vasculature may be computed with a so-called "minimal path" technique such as Fast-Marching. In this technique, starting from the reference vasculature, for every position in the vasculature represented in the image, the minimal path for that position is the one that leads from the reference position to this position while covering the shortest geodesic distance. The geodesic distance over a path can be defined as the cumulated potential values along that path, where the potential value at any pixel is for instance a function of the vesselness. The higher the vesselness, the lower the potential value.

By way of an example, the potential value can be set to a small epsilon value, $\varepsilon$, at any point belonging to the vessel tree and to a large value, say 20 times epsilon, at all the points that do not belong to the vasculature. For a reference position $P_{ref}$ and any point $P_d$ in the vasculature, the minimal path $C^*(P_{ref}, P_d)$ will link $P_{ref}$ to $P_d$ while achieving the smallest possible sum of all the potential values encountered on that path. To every path $C(P_{ref}, P_d)$ linking $P_{ref}$ and $P_d$ corresponds a number $A(C(P_{ref}, P_d))$ equal to the sum of the potential values encountered on that path. The minimal path $C^*(P_{ref}, P_d)$ corresponds to the smallest possible value of A among all the possible paths from $P_{ref}$ to $P_d$. As mentioned above, this minimal path can be found efficiently using the Fast-Marching algorithm. The longest vessel in the angiogram has been shown to provide a reliable estimate of the transit time, and the transit velocity. This is because it is robust to changes in the topology of the vasculature, and does not overestimate the velocity in the case the contrast agent progresses simultaneously in two parallel branches.

Having tracked the position of the front in the images in the temporal sequence, proximal and distal position $150_p$ and $150_d$, are defined in the vessel 140, and the transit time, or the transit velocity, is calculated based on the tracked positions of the front in relation to the proximal position $150_p$ and the distal position $150_d$. In general, the proximal and distal positions may be defined automatically using feature detection techniques, or a trained neural network, or manually, i.e. the positions may be defined in response to user input. Various examples of these operations are described in the examples below.

In one example, the transit time, or the transit velocity, for a front, is calculated based on the time taken by the front, or the average speed of the front, in passing between a proximal position in an earlier image in the temporal sequence and a distal position in a later image in the temporal sequence. In this example, the proximal and distal positions are defined by the positions of the fronts in the earlier and later images respectively. The positions of the fronts are defined by selecting images from the temporal sequence in which the fronts are in the desired proximal and distal positions. The proximal and distal positions may be defined in locations such as those described above, i.e. the proximal position may be proximal to the ostium of the vessel, and the distal position may be a position in the "distal two-thirds" of the vessel, or the most distal position in the vessel in the image. Images in which the fronts are in these desired positions may be selected automatically using feature detection techniques, or a trained neural network, or manually, i.e. the positions may be defined in response to user input.

In this example, the transit time is then calculated by subtracting the time of the earlier image from the time of the later image. The transit time is therefore calculated based on the tracked positions of the front in relation to the proximal position $150_p$ and the distal position $150_d$.

In this example, the transit velocity may be calculated using the transit time by dividing a distance traversed by the front between the proximal position in the earlier image, and the distal position in the later image, by the calculated transit time. In order to calculate this distance, the proximal and distal positions $150_p$, $150_d$, may be mapped to a common image by registering the earlier image to the later image, or vice versa. Known image registration techniques may be used to perform this registration.

In general the transit velocity may be calculated in terms of any reference unit of length. The velocity may for example be calculated in real-world units such as centimetres per second, or in terms of another reference unit of length, such as pixels per second. In the later operation S130, the values of some blood flow parameters may be calculated using a ratio of two velocities over the same path length, and in such cases the distances that are used to calculate the velocities cancel one another when calculating the ratio. In such cases the velocity may be calculated in terms of a reference unit such as pixels per second.

If a velocity measurement is desired in terms of a real-world dimension, the velocity may be evaluated by applying a known scaling between pixels in the angiographic image and the real-world dimension, e.g. a specified amount of "centimetres per pixel". The scaling may be known from a calibration of the imaging system that generated the angiographic image. Such a calibration may be known for the current geometry of the imaging system. The calibration may alternatively be computed based on the geometry of the imaging system. For instance, if the imaging system is an X-ray projection imaging system, the calibration may be computed using parameters such as the relative separation between the X-ray source, vessel, and X-ray detector, and a linear dimension of the X-ray detector. Alternatively, such a calibration may be determined based on the known dimensions of a feature that is included in the angiographic images. The scaling operation described above may be performed in combination with a foreshortening correction to account for out-of-plane deviations in the shape of the vessel. The amount of foreshortening may be calculated based on an assumed shape of the vessel 140, and geometry of the imaging system.

The calculation of a transit time, or a transit velocity in accordance with this example has the benefit of being relatively fast to execute. However, it can lead to errors in the calculated transit time due to the limited number of measurements: if the images used to compute the transit times are selected incorrectly (e.g. if the front is difficult to identify clearly), this directly lead to errors in the measurements.

In another example, the transit velocity is calculated based on the distances that are traversed by the contrast agent front along the vessel from a reference position in each of multiple images in the temporal sequence. In this example, the operation of calculating a transit velocity based on the tracked positions comprises:

- calculating, for the tracked positions, a corresponding distance 190 traversed by the front along the vessel 140 from a reference position in the vessel;
- calculating an average velocity of the front between the proximal position $150_p$ and the distal position $150_d$, the average velocity being calculated so as to minimise, for a plurality of images in the temporal sequence in which the front is located between the proximal position $150_p$ and the distal position $150_d$, a difference between the distance traversed by the front along the vessel 140 in the image and a distance traversed by the front along the vessel at the average velocity; and
- using the average velocity as the transit velocity.

This example is described with reference to Fig. 4, which is a graph illustrating an example of the distance, Length, 190, traversed by a front from a reference position in the vessel at the times, Frame #, of corresponding angiographic images, in accordance with some aspects of the present disclosure. The horizontal axis of the graph illustrated in Fig. 4 represents the times of angiographic images in a temporal sequence via their Frame number. The vertical axis of the graph illustrated in Fig. 4 represents the distance traversed by a front from a reference position in the vessel, in each of the angiographic images in the temporal sequence. The temporal sequence of images that was used to generate the graph illustrated in Fig. 4 was generated with the vessel in the basal state, and the graph is therefore used to calculate the transit velocity in the basal state. A similar graph to that illustrated in Fig. 4 may be obtained from a temporal sequence of images representing the vessel in the hyperemic state, and thus used to calculate a transit velocity for the vessel in the hyperemic state.

The graph illustrated in Fig. 4 may be obtained by tracking the position of the contrast agent front in the images in the temporal sequence using the techniques described above. For each image, i.e. Frame, in the temporal sequence, a distance, i.e. the "Length", is measured between a reference position in the vessel, and the contrast agent front. In the example illustrated in Fig. 4, the reference position is the ostium of the LAD. This results in a series of vessel lengths $[L_0 = 0, L_1, L_2, ... L_n]$ between the reference point and the front, at the times of the corresponding images $[T_0, T_1, T_2, ...T_n]$. In general, any position in the vessel may be chosen as the reference position. Thus, as described above, an anatomical landmark, or an interventional device landmark, may be chosen as the reference position. For example, the ostium of the vessel, or a bifurcation in the vessel, or a position on the injection catheter, may be chosen as the reference position. The reference position may be chosen as the proximal position $150_p$.

In the example illustrated in Fig. 4, the Length is approximately zero centimetres for the period 0 < Frame number (i.e. time) < 6. During this period, the contrast agent front has not yet passed the reference position. Variations in the Length within this period represent measurement noise in the operation of tracking the position of the contrast agent front. The contrast agent front passes the reference position at approximately Frame number (i.e. time) = 6, and after this time, the Length increases approximately linearly until approximately Frame number (i.e. time) = 15. At Frame number (i.e. time) = 15, the contrast agent has completely filled the vessel, and in the subsequent angiographic images for Frame number (i.e. time) > 15, the Length remains approximately constant at Length = 8 centimetres. Variations in the Length after Frame number (i.e. time) = 15 represent measurement noise and are caused by variations in the measurement of the Length in the filled vessel in subsequent images.

In the example illustrated in Fig. 4, the Length is calculated in centimetres. However, it is noted that the Length may in general be calculated in any reference unit. As described above, some blood flow parameters are evaluated by calculating a ratio between two velocities, and in such cases the distances that are used to calculate the velocities cancel one another when calculating the ratio. If required, the Length in the graph illustrated in Fig. 4 may be calculated in real-world units such as centimetres by applying a scaling, as described above. The scaling operation described above may be performed in combination with a foreshortening correction to account for out-of-plane deviations in the shape of the vessel, as also described above.

An average velocity of the front between the proximal position and the distal position is then calculated. The average velocity is calculated so as to minimise, for a plurality of images in the temporal sequence in which the front is located between the proximal position and the distal position, a difference between the distance traversed by the front along the vessel in the image and a distance traversed by the front along the vessel at the average velocity. This may be calculated by performing an optimisation procedure wherein the value the transit velocity, $V$, is set so as to minimise a function of the errors $[(L_0 - V.T_0), (L_1 - V.T_1) ... (L_n - V.T_n)]$, for a plurality of images in the temporal sequence in which the front is located between the proximal position and the distal position.

In the example illustrated in Fig. 4, this operation is performed using images in the temporal sequence in which the velocity is in the linear range, i.e. between approximately Frame number (i.e. time) = 6 and Frame number (i.e. time) =15. The start, and the end, of this time period then define the proximal and distal positions of the front, respectively. If the start, or the end of this time period coincide with the Frame number (i.e. time) of an angiographic image, the corresponding position is a real position that is identifiable as the position of the front in the corresponding image. Thus, if the transit time is deemed to start at Frame number (i.e. time) = 6, and the transit time is deemed to end at Frame number (i.e. time) = 15, then the corresponding proximal and distal positions are real positions that are identifiable as the positions of the front in the corresponding images. Thus, as described above, in this example, the transit velocity is calculated based on the tracked positions of the front in relation to the proximal position $150_p$ and the distal position $150_d$, and in this example the transit velocity is the average velocity between the proximal and distal positions.

In another example, the transit velocity is calculated based on the instantaneous velocities of the front in the images in the temporal sequence whilst the front is between the proximal and distal positions in the vessel. In this example, the operation of calculating the transit velocity based on the tracked positions includes:

- calculating, for each of a plurality of images in the temporal sequence in which the front is between the proximal position $150_p$ and the distal position $150_d$, an instantaneous velocity of the front, the instantaneous velocity of the front being calculated based on a distance traversed by the front along the vessel 140 between successive images in the temporal sequence and a time interval between the successive images; and
- calculating the transit velocity based on the instantaneous velocities.

With reference to Fig. 4, in this example, instantaneous velocities of the front may be calculated for each image in the temporal sequence within the linear range, i.e. from Frame number (i.e. time) = 6 until Frame number (i.e. time) = 15. The positions of the front in the first and last images in the selected time period define the proximal and distal positions respectively. The instantaneous velocity of the front may be calculated for each image within the linear region by determining the gradient of $(L, - L_{i-1})/\Delta T$ for images, $i$, within the linear range and wherein $\Delta T$ is the time interval between images. The instantaneous velocities may then be averaged to provide the transit velocity. Thus, in this example, the transit velocity is calculated based on the tracked positions of the front in relation to the proximal position $150_p$ and the distal position $150_d$.

In another example, the transit velocity is calculated by fitting a straight line to the Length measurements in the linear range of the graph illustrated in Fig. 4, i.e. from Frame number (i.e. time) = 6 until Frame number (i.e. time) = 15. The gradient of this line represents an average velocity, and this may also be used as the transit velocity. This example is also illustrated in Fig. 4 as the line labelled "Trapezoid fitting". The positions of the front at the start and the end of this time period again define the proximal and distal positions respectively. It is noted that the start, or the end, of the transit time may correspond to the time of an angiographic image, or they may be correspond to a time that is between the times of two angiographic images. In the former case, the corresponding proximal, or distal, position is a real position in the vessel that is identifiable in the corresponding angiographic image. In the latter case, the corresponding proximal, or distal, position is defined by a virtual position in the vessel that is located between the real positions of the front in the vessel in the images either side of the start, or end, of the transit time. Thus, as described above, in this example, the transit velocity is calculated based on the tracked positions of the front in relation to the proximal position $150_p$ and the distal position $150_d$, and in this example the transit velocity is the average velocity between the proximal and distal positions.

Referring back to Fig. 2, in the operation S130, the one or more processors of the system 100, output S 130 a value of one or more blood flow parameters 160 for the vessel 140. The value of the one or more blood flow parameters is calculated based on the values of the transit times, or the values of the transit velocities.

In the operation S130, the value of one or more blood flow parameters such as the CFR, or the MFR, or the IMR, are calculated. The value(s) of these parameters may be calculated using the Equations 1-4 above. In the example of parameters such as the CFR, and the MFR, and which are evaluated based on the measurements of the transit time or transit velocity in both a first haemodynamic state, and a second haemodynamic state, a value for a single blood flow parameter may be outputted in the operation S130. Parameters such as the CFR, and the MFR, express the relative change between the two states. In the example of parameters such as the IMR, and which are typically evaluated based on the measurements of the transit time or transit velocity in only one state, i.e. the first haemodynamic state, or the second haemodynamic state, a value for the blood flow parameter may be outputted for each of the two states in the operation S 130. The values of such blood flow parameters for each of the two states may also be combined into a single blood flow parameter. For example a ratio may be evaluated using their individual values in order to express the relative change between the two states in a single parameter. Thus, in this case a value of a single, combined, blood flow parameter may be outputted in the operation S130.

The value of the blood flow parameter(s) may be outputted in the operation S130 in any human-intelligible format. For instance, the value of the blood flow parameter(s) may be outputted visually, or audially. In the former case, the blood flow parameters may be outputted in any format, including numerically, or graphically. With reference to the system illustrated in Fig. 1, the value of the blood flow parameter(s) may be outputted to a display, such as the monitor 230, for example. The value of the blood flow parameter(s) may also be outputted to a computer-readable storage medium in the operation S130.

Referring back to Fig. 2, in the operation S140, the one or more processors of the system 100, output S140 a first angiogram $170_1$, and a second angiogram $170_2$. The first angiogram, and the second angiogram are generated from the angiographic image data 120, and depict the motion of the fronts $130_1$, $130_2$ along the vessel 140 in the first haemodynamic state, and the second haemodynamic state, respectively. Moreover, the motion of the fronts $130_1$, $130_2$ depicted in the first angiogram $170_1$, and the second angiogram $170_2$, is synchronised such that the fronts in both angiograms simultaneously leave the proximal position $150_p$ in the vessel.

The first angiogram, and the second angiogram may be outputted to a display, such as the monitor 230 illustrated in Fig. 1, for example. The angiograms may also be outputted to a computer-readable storage medium. As described above, the angiograms provide insights into the angiographic measurement of the transit times, or transit velocities, that underly the calculated value of the blood flow parameter(s), and these insights help to improve a physician's confidence in the calculated value(s).

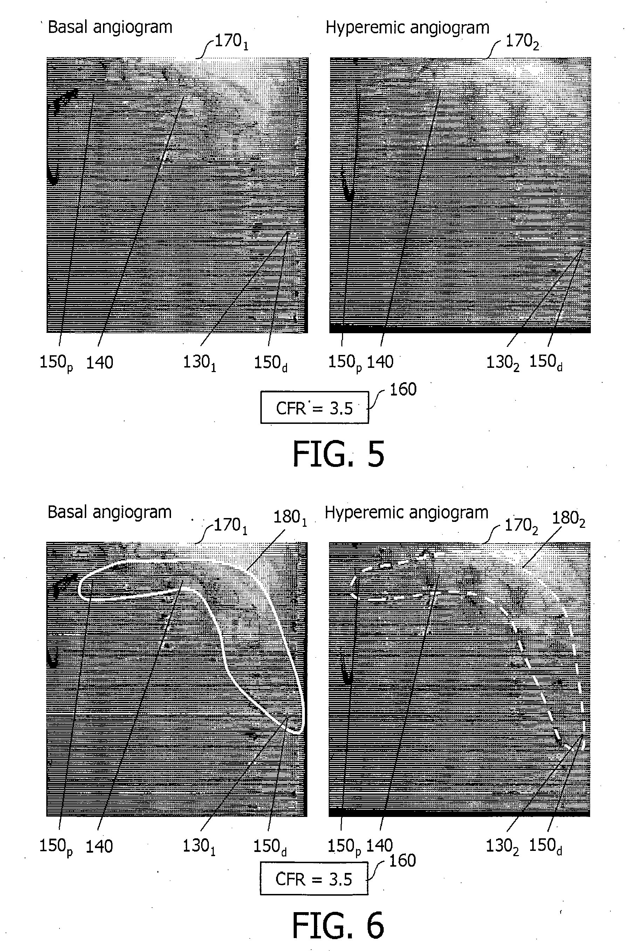

An example of the angiograms that may be outputted in the operation S140 is illustrated in Fig. 5, which is an example of a first angiogram $170_1$ and a second angiogram $170_2$ depicting the motion of a front $130_1$, $130_2$ of an injected contrast agent along a vessel 140 in a first haemodynamic state, and a second haemodynamic state, respectively, in accordance with some aspects of the present disclosure. Fig. 5 also illustrates an example of a corresponding value of a blood flow parameter 160, in this case the CFR, and which is outputted in the operation S130 described above. The motion of the fronts $130_1$, $130_2$ in the angiograms $170_1$ and $170_2$ illustrated in Fig. 5 is synchronised such that the fronts in both angiograms simultaneously leave the proximal position $150_p$ in the vessel. Consequently, a physician may observe from the relative motion of the fronts along the vessel that the measurements of the blood flow that underly the calculated value of the blood flow parameter(s) are physiologically sound. Thus, the angiograms provide insights into the calculated value of the blood flow parameter(s) that help to improve a physician's confidence in their value(s). In the example illustrated in Fig. 5, both of the fronts $130_1$ and $130_2$ have reached the distal position $150_d$ in the vessel 140.

In one example, the angiograms depict the motion of fronts in multiple vessels, and a value of one or more blood flow parameter(s) is calculated for each of the vessels. In this example, the system comprises one or more processors 110 configured to:

- receive S110 angiographic image data 120 representing a motion of a front $130_1$, $130_2$ of an injected contrast agent along each of a plurality of different vessels in each of a first haemodynamic state, and a second haemodynamic state;
- analyse S120 the angiographic image data 120 to determine a transit time, or a transit velocity, for each vessel in each state, the transit time in each state being calculated based on a time taken for the front to pass between a proximal position $150_p$ in the vessel, and a distal position $150_d$ in the vessel, and the transit velocity in each state being calculated based on one or more distances traversed by the front between the proximal position $150_p$ in the vessel, and the distal position $150_d$ in the vessel, and one or more corresponding time intervals;
- output S130 a value of one or more blood flow parameters 160 for each vessel, the value of the one or more blood flow parameters being calculated based on the values of the transit times, or the values of the transit velocities for the corresponding vessel; and
- output S140 a first angiogram $170_1$, and a second angiogram $170_2$, the first angiogram, and the second angiogram being generated from the angiographic image data 120 and depicting the motion of the fronts $130_1$, $130_2$ along the vessels in the first haemodynamic state, and the second haemodynamic state, respectively; and
- wherein the motion of the fronts $130_1$, $130_2$ depicted in the first angiogram $170_1$, and the second angiogram $170_2$, is synchronised such that the fronts in both angiograms simultaneously leave the proximal position $150_p$ in each vessel.

In this example, the one or more blood flow parameters are evaluated for each of the multiple vessels. Blood flow parameters may have different values in different vessels in the vasculature. By evaluating the blood flow parameter(s) for each of the multiple vessels and providing a graphical representation 170 that depicts the motion of the fronts $130_1$, $130_2$ between the proximal position $150_p$ and the distal position $150_d$, $150_d'$ in each of the vessels 140, 140', a physician may determine that the depicted motion of the fronts that is used to calculate the values of the blood flow parameters, fits with their expectation for the different vessels. Thus, it may help to improve a physician's confidence in the calculated value of the blood flow parameter(s).

In another example, the angiograms repetitively depict the motion of the fronts along the vessel. In this example, the first angiogram $170_1$, and the second angiogram $170_2$, repetitively depict the motion of the fronts $130_1$, $130_2$ along the vessel 140 in the first haemodynamic state, and the second haemodynamic state, respectively. Moreover, the motion of the fronts $130_1$, $130_2$ depicted in the first angiogram $170_1$, and the second angiogram $170_2$, is synchronised such that in each repetition the fronts in both angiograms simultaneously leave the proximal position $150_p$ in the vessel 140.

The repetitive, or "looped", display of the motion of the fronts provides a physician with the same information multiple times, allowing the physician to see details that are missed on a graphical representation that provides only a single transit of the fronts. This may further improve a physician's confidence in the calculated value of the blood flow parameter(s).

In this example, a delay may be provided between each repetition. The delay may occur prior to each repetition, wherein during the delay the fronts $130_1$, $130_2$ in both angiograms $170_1$, $170_2$ are depicted at the proximal position $150_p$. in the vessel 140. Alternatively, the delay may occur after each repetition, wherein during the delay the fronts $130_1$, $130_2$ in both angiograms $170_1$, $170_2$ are depicted at the distal position $150_d$ in the vessel 140.

In another example, markers are overlaid on the fronts in the angiograms. In this example, the one or more processors 110 are configured to overlay each of the first angiogram $170_1$, and the second angiogram $170_2$ with a marker depicting the front $130_1$, $130_2$ of the injected contrast agent in the respective angiogram.

In this example, the position of the marker corresponds to the tracked position of the front. The tracked positions of the fronts are determined using the vessel tracking techniques described above. The tracked positions are the positions of the fronts that are used to calculate the transit times, or the transit velocities. By providing such markers, a physician's attention is brought to the positions of the fronts that are used to calculate the values of the transit times, or transit velocities, or in other words their attention is brought to the measurements of the blood flow that underly the calculated value of the blood flow parameter(s). Thus, by indicating the positions of the fronts, the markers may further improve a physician's confidence in the calculated value of the blood flow parameter(s).

Various markers may be used to depict the fronts $130_1$, $130_2$ of the injected contrast agent in the angiograms. In general the markers may have various shapes, colours, or intensities that draw attention to the position of the front. For example, the markers may have an intensity or colour that contrasts with an intensity or colour of the vessel in the angiogram, or with a background intensity or colour in the angiogram. The markers may also be provided with different intensities, or colours, or shapes, to enable a distinction between the fronts $130_1$, $130_2$. Different types of markers may also be used to depict the fronts $130_1$, $130_2$.

In another example, the one or more processors 110 are configured to track a path of the injected contrast agent in the temporal sequence of images; and overlay each of the first angiogram $170_1$, and the second angiogram $170_2$ with a trace depicting the tracked path of the injected contrast agent in the respective angiogram.

In this example, the path of the contrast agent may be tracked using the vessel tracking techniques described above. The trace may depict the centerline of the tracked path of the injected contrast agent in the temporal sequence of images, and a distal end of the path may correspond to the tracked position of the front $130_1$, $130_2$ in the temporal sequence of images.

By indicating the tracked path of the injected contrast agent, this example provides a further insight into the measurements of the blood flow that underly the calculated values of the transit times, or transit velocities, or in other words the measurements of the blood flow that underly the calculated value of the blood flow parameter(s). Thus, by indicating the positions of the fronts, the markers may further improve a physician's confidence in the calculated value of the blood flow parameter(s). By indicating the position of the centerline, a physician may observe the correspondence of the centerline with the vessel, indicating the absence of errors in tracking the vessel in the temporal sequence of images.

In this example, the trace depicting the tracked path of the injected contrast agent in the respective angiogram may have an intensity or a colour that contrasts with that of a background intensity or colour in the angiogram. For example, the trace may be depicted in colour, whereas the vessel may be depicted in dark contrast. If the trace depicts a centerline of the tracked path of the injected contrast agent, the centerline may be depicted in light contrast, thereby contrasting with a vessel in dark contrast, for example.

In another example, a portion of the vessel that includes the proximal position and the distal position is identified in the angiographic images. In this example, the one or more processors 110 are configured identify a portion of the vessel 140 comprising the proximal position $150_p$ and the distal position $150_d$ in each of the first angiogram $170_1$, and the second angiogram $170_2$.

By identifying this portion of the vessel, a physician's attention is brought to the portion of the vessel for which the value of the blood flow parameter(s) is calculated. The vessel may undergo motion in the angiograms, and identifying this portion of the vessel helps to maintain the physician's focus on the relevant part of the vessel. The vessel motion may arise from various sources, including cardiac motion, and patient motion. The portion of the vessel may be identified using various techniques. For instance, the intensity, or the colour, of the vessel, may be adjusted in relation to the background intensity in the angiogram, in order to highlight the relevant portion of the vessel.

In a related example, the one or more processors 110 are configured to identify the portion of the vessel 140 by providing an overlay region $180_1$, $180_2$ encompassing a length of the vessel between the proximal position $150_p$ and the distal position $150_d$ in each of the first angiogram $170_1$, and the second angiogram $170_2$. In this example, the one or more processors 110 are configured to determine a shape of the overlay region by tracking a path of the injected contrast agent in the temporal sequence of images; and defining a shape of the overlay region such that the shape of the overlay region encompasses the tracked path of the injected contrast agent between the proximal position $150_p$ and the distal position $150_d$ in the temporal sequence of images.

This example is illustrated in Fig. 6, which is a second example of a first angiogram $170_1$ and a second angiogram $170_2$ depicting the motion of a front $130_1$, $130_2$ of an injected contrast agent along a vessel 140 in a first haemodynamic state, and a second haemodynamic state, respectively, in accordance with some aspects of the present disclosure. The motion of the fronts $130_1$, $130_2$ depicted in the first angiogram $170_1$, and the second angiogram $170_2$, illustrated in Fig. 6, is synchronised such that the fronts in both angiograms simultaneously leave the proximal position $150_p$ in the vessel.

The example illustrated in Fig. 6 is similar to the example illustrated in Fig. 5, and items in Fig, 6 with the same labels as those illustrated in Fig. 5 refer to the same features. In the example illustrated in Fig. 6, both of the fronts $130_1$ and $130_2$ have reached the distal position $150_d$ in the vessel 140. In contrast to Fig. 5, the angiograms $170_1$, and $170_1$ include overlay regions $180_1$, and $180_2$, respectively. The shapes of the overlay regions $180_1$, $180_2$ encompass the tracked path of the injected contrast agent between the proximal position $150_p$ and the distal position $150_d$ in the corresponding angiogram. The tracked path of the injected contrast agent may be determined using the vessel tracking techniques described above. The shapes of the overlay regions $180_1$, $180_2$ encompass motion of the vessel in the angiograms over time.

In a related example, the overlay region $180_1$, $180_2$ in each of the first angiogram $170_1$, and the second angiogram $170_2$ comprises a fixed shape. The fixed shape may be determined by e.g. determining the spatial limits to the extent of the tracked path of the injected contrast agent in the angiograms $170_1$, and $170_2$, and providing a shape that encompasses these limits. The overlay region $180_1$, $180_2$ in each of the first angiogram $170_1$, and the second angiogram $170_2$ may alternatively comprise a temporally-varying shape, and wherein the temporally-varying shape is determined for a current image in each angiogram based on the tracked path of the injected contrast agent in the corresponding image in the temporal sequence of images and zero or more earlier or later images in the temporal sequence of images. Thus, in this case the shape may for example extend along the vessel 140 over time in accordance with the progression of the corresponding front.

It is noted that in the examples described above, the system 100 may also include one or more of: an angiographic imaging system for providing the angiographic image data 120, such as for example the projection X-ray imaging system 210 illustrated in Fig. 1; an injector 220 for injecting the contrast agent; a display, such as the monitor 230 illustrated in Fig. 1, for displaying the outputted value of the blood flow parameter 160, the graphical representation 170 of the vessel 140, the angiographic image data, and so forth; a patient bed 220; and a user input device (not illustrated in Fig. 1) that is configured to receive user input relating to the operations performed by the one or more processors of the system 100, such as a keyboard, a mouse, a touchscreen, and so forth.

In another example, a computer-implemented method of evaluating blood flow parameters, is provided. The method comprises:

- receiving S110 angiographic image data 120 representing a motion of a front $130_1$, $130_2$ of an injected contrast agent along a vessel 140 in each of a first haemodynamic state, and a second haemodynamic state;
- analysing S120 the angiographic image data 120 to determine a transit time, or a transit velocity, for the vessel 140 in each state, the transit time in each state being calculated based on a time taken for the front to pass between a proximal position $150_p$ in the vessel, and a distal position $150_d$ in the vessel, and the transit velocity in each state being calculated based on one or more distances traversed by the front between the proximal position $150_p$ in the vessel, and the distal position $150_d$ in the vessel, and one or more corresponding time intervals;
- outputting S 130 a value of one or more blood flow parameters 160 for the vessel 140, the value of the one or more blood flow parameters being calculated based on the values of the transit times, or the values of the transit velocities; and
- outputting S140 a first angiogram $170_1$, and a second angiogram $170_2$, the first angiogram, and the second angiogram being generated from the angiographic image data 120 and depicting the motion of the fronts $130_1$, $130_2$ along the vessel 140 in the first haemodynamic state, and the second haemodynamic state, respectively; and
- wherein the motion of the fronts $130_1$, $130_2$ depicted in the first angiogram $170_1$, and the second angiogram $170_2$, is synchronised such that the fronts in both angiograms simultaneously leave the proximal position $150_p$ in the vessel.

In another example, a computer program product, is provided. The computer program product comprises instructions which when executed by one or more processors, cause the one or more processors to carry out a method of evaluating blood flow parameters. The method comprises:

- receiving S110 angiographic image data 120 representing a motion of a front $130_1$, $130_2$ of an injected contrast agent along a vessel 140 in each of a first haemodynamic state, and a second haemodynamic state;
- analysing S120 the angiographic image data 120 to determine a transit time, or a transit velocity, for the vessel 140 in each state, the transit time in each state being calculated based on a time taken for the front to pass between a proximal position $150_p$ in the vessel, and a distal position $150_d$ in the vessel, and the transit velocity in each state being calculated based on one or more distances traversed by the front between the proximal position $150_p$ in the vessel, and the distal position $150_d$ in the vessel, and one or more corresponding time intervals;
- outputting S 130 a value of one or more blood flow parameters 160 for the vessel 140, the value of the one or more blood flow parameters being calculated based on the values of the transit times, or the values of the transit velocities; and
- outputting S140 a first angiogram $170_1$, and a second angiogram $170_2$, the first angiogram, and the second angiogram being generated from the angiographic image data 120 and depicting the motion of the fronts $130_1$, $130_2$ along the vessel 140 in the first haemodynamic state, and the second haemodynamic state, respectively; and
- wherein the motion of the fronts $130_1$, $130_2$ depicted in the first angiogram $170_1$, and the second angiogram $170_2$, is synchronised such that the fronts in both angiograms simultaneously leave the proximal position $150_p$ in the vessel.

Embodiments of the methods or computer-implemented methods may comprise the steps that are executed by the one or more processors according to any of the embodiments of the systems.

The above examples are to be understood as illustrative of the present disclosure, and not restrictive. Further examples are also contemplated. For instance, the examples described in relation to the system 100, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for evaluating blood flow parameters, the system comprising one or more processors (110) configured to:

   receive (S110) angiographic image data (120) representing a motion of a front ($130_1$, $130_2$) of an injected contrast agent along a vessel (140) in each of a first haemodynamic state and a second haemodynamic state;
   analyse (S120) the angiographic image data (120) to determine a transit time or a transit velocity, for the vessel (140) in each state, the transit time in each state being calculated based on a time taken for the front to pass between a proximal position ($150_p$) in the vessel and a distal position ($150_d$) in the vessel, and the transit velocity in each state being calculated based on one or more distances traversed by the front between the proximal position ($150_p$) in the vessel and the distal position ($150_d$) in the vessel, and one or more corresponding time intervals;
   output (S130) a value of one or more blood flow parameters (160) for the vessel (140), the value of the one or more blood flow parameters being calculated based on the values of the transit times or the values of the transit velocities; and
   output (S140) a first angiogram ($170_1$) and a second angiogram ($170_2$), the first angiogram and the second angiogram being generated from the angiographic image data (120) and depicting the motion of the fronts ($130_1$, $130_2$) along the vessel (140) in the first haemodynamic state and the second haemodynamic state, respectively; and
   wherein the motion of the fronts ($130_1$, $130_2$) depicted in the first angiogram ($170_1$), and the second angiogram ($170_2$), is synchronised such that the fronts in both angiograms simultaneously leave the proximal position ($150_p$) in the vessel.

2. The system according to claim 1, wherein the first angiogram ($170_1$) and the second angiogram ($170_2$) repetitively depict the motion of the fronts ($130_1$, $130_2$) along the vessel (140) in the first haemodynamic state and the second haemodynamic state, respectively;

wherein the motion of the fronts ($130_1$, $130_2$) depicted in the first angiogram ($170_1$) and the second angiogram ($170_2$), is synchronised such that in each repetition the fronts in both angiograms simultaneously leave the proximal position ($150_p$) in the vessel (140).

3. The system according to claim 2, wherein the first angiogram ($170_1$) and the second angiogram ($170_2$) repetitively depict the motion of the fronts ($130_1$, $130_2$) along the vessel (140) with a delay between each repetition.

4. The system according to claim 3, wherein the delay occurs prior to each repetition, and wherein during the delay the fronts ($130_1$, $130_2$) in both angiograms ($170_1$, $170_2$) are depicted at the proximal position ($150_p$) in the vessel (140).

5. The system according to claim 3, wherein the delay occurs after each repetition, and wherein during the delay the fronts ($130i$, $130_2$) in both angiograms ($170i$, $170_2$) are depicted at the distal position ($150_d$) in the vessel (140).

6. The system according to any previous claim, wherein the received angiographic image data (120) comprises a temporal sequence of images representing the motion of the front ($130_1$, $130_2$) of the injected contrast agent along the vessel (140) in each of the first haemodynamic state and the second haemodynamic state, and wherein the one or more processors (110) are configured to:

   generate the first angiogram ($170_1$), and the second angiogram ($170_2$), from the temporal sequence of images representing the first haemodynamic state and the second haemodynamic state, respectively; and
   analyse the angiographic image data (120) to determine the transit time or the transit velocity for the vessel (140) in each of the first haemodynamic state and the second haemodynamic state, by:

   tracking a position of the front ($130_1$, $130_2$) in the temporal sequence of images;
   defining the proximal position ($150_p$) and the distal position ($150_d$) in the vessel (140); and
   calculating the transit time or the transit velocity, based on the tracked positions of the front in relation to the proximal position ($150_p$) and the distal position ($150_d$).

7. The system according to claim 6, wherein the calculating the transit velocity based on the tracked positions comprises:

   calculating, for the tracked positions, a corresponding distance (190) traversed by the front ($130_1$, $130_2$) along the vessel (140) from a reference position in the vessel;
   calculating an average velocity of the front ($130_1$, $130_2$) between the proximal position ($150_p$) and the distal position ($150_d$), the average velocity being calculated so as to minimise, for a plurality of images in the temporal sequence in which the front is located between the proximal position and the distal position, a difference between the distance traversed by the front along the vessel (140) in the image and a distance traversed by the front along the vessel at the average velocity; and
   using the average velocity as the transit velocity.

8. The system according to claim 6 or claim 7, wherein the one or more processors (110) are further configured to:
   overlay each of the first angiogram ($170_1$), and the second angiogram ($170_2$) with a marker depicting the front ($130_1$, $130_2$) of the injected contrast agent in the respective angiogram.

9. The system according to any one of claims 6 - 8, wherein the one or more processors (110) are further configured to:

   track a path of the injected contrast agent in the temporal sequence of images; and
   overlay each of the first angiogram ($170_1$) and the second angiogram ($170_2$) with a trace depicting the tracked path of the injected contrast agent in the respective angiogram.

10. The system according to claim 9, wherein the trace depicts the centerline of the tracked path of the injected contrast agent in the temporal sequence of images, and wherein a distal end of the path corresponds to the tracked position of the front ($130_1$, $130_2$) in the temporal sequence of images.

11. The system according to any previous claim, wherein the proximal position ($150_p$) corresponds to an anatomical landmark, or an interventional device landmark.

12. The system according to any one of claims 6 - 11, wherein the one or more processors (110) are further configured identify a portion of the vessel (140) comprising the proximal position ($150_p$) and the distal position ($150_d$) in each of the first angiogram ($170_1$) and the second angiogram ($170_2$).

13. The system according to claim 12, wherein the one or more processors (110) are configured to identify the portion of the vessel (140) by providing an overlay region ($180_1$, $180_2$) encompassing a length of the vessel between the proximal position ($150_p$) and the distal position ($150_d$) in each of the first angiogram ($170_1$) and the second angiogram ($170_2$); and
wherein the one or more processors (110) are configured to determine a shape of the overlay region by:

> tracking a path of the injected contrast agent in the temporal sequence of images; and
> defining a shape of the overlay region such that the shape of the overlay region encompasses the tracked path of the injected contrast agent between the proximal position ($150_p$) and the distal position ($150_d$) in the temporal sequence of images.

14. The system according to claim 13, wherein:

> the overlay region ($180_1$, $180_2$) in each of the first angiogram ($170_1$), and the second angiogram ($170_2$) comprises a fixed shape;
> or
> wherein the overlay region ($180_1$, $180_2$) in each of the first angiogram ($170_1$), and the second angiogram ($170_2$) comprises a temporally-varying shape, and wherein the temporally-varying shape is determined for a current image in each angiogram based on the tracked path of the injected contrast agent in the corresponding image in the temporal sequence of images and zero or more earlier or later images in the temporal sequence of images.

15. The system according to any previous claim, wherein the one or more blood flow parameters (160) include one or more of: the coronary flow reserve, CFR, or the index of microvascular resistance, IMR, or the maximal flow ratio, MFR.'

FIG. 1

FIG. 2

FIG. 3

FIG. 4

Basal angiogram

Hyperemic angiogram

$150_p$  140  $130_1$  $150_d$  $150_p$ 140  $130_2$  $150_d$

CFR = 3.5  160

# FIG. 5

Basal angiogram

Hyperemic angiogram

$150_p$  140  $130_1$  $150_d$  $150_p$ 140  $130_2$  $150_d$

CFR = 3.5  160

# FIG. 6

Europäisches
Patentamt

European
Patent Office

Office européen
des brevets

# EUROPEAN SEARCH REPORT

Application Number

EP 22 29 0080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/089097 A1 (OHISHI SATORU [JP] ET AL) 31 March 2016 (2016-03-31) | 1,6, 8-11,15 | INV.<br>A61B6/00<br>A61B5/0285 |
| Y | * paragraphs [0136], [0160], [0036] * | 12-14 | G06T7/00 |
| X | US 2008/319309 A1 (BREDNO JOERG [DE] ET AL) 25 December 2008 (2008-12-25) | 1-11,15 | ADD. |
| Y | * paragraphs [0060], [0052], [0056] * | 12-14 | A61B5/02 |
| Y | EP 4 020 390 A1 (KONINKLIJKE PHILIPS NV [NL]) 29 June 2022 (2022-06-29)<br>* paragraph [0136] * | 12-14 | |

TECHNICAL FIELDS
SEARCHED        (IPC)

A61B
G06T

The present search report has been drawn up for all claims

| Place of search | Date of completion of the search | Examiner |
|---|---|---|
| Munich | 4 May 2023 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 29 0080

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2023

| Patent document cited in search report | | Publication date | Patent family member(s) | | Publication date |
|---|---|---|---|---|---|
| US 2016089097 | A1 | 31-03-2016 | JP | 6537797 B2 | 03-07-2019 |
| | | | JP | 2016067568 A | 09-05-2016 |
| | | | US | 2016089097 A1 | 31-03-2016 |
| US 2008319309 | A1 | 25-12-2008 | CN | 101330872 A | 24-12-2008 |
| | | | EP | 1962688 A2 | 03-09-2008 |
| | | | JP | 5236489 B2 | 17-07-2013 |
| | | | JP | 2009519082 A | 14-05-2009 |
| | | | US | 2008319309 A1 | 25-12-2008 |
| | | | WO | 2007069166 A2 | 21-06-2007 |
| EP 4020390 | A1 | 29-06-2022 | EP | 4020390 A1 | 29-06-2022 |
| | | | WO | 2022136204 A1 | 30-06-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PIJLS, N. H. et al.** Concept of maximal flow ratio for immediate evaluation of percutaneous transluminal coronary angioplasty result by videodensitometry. *Circulation,* 1991, vol. 83, 854-865

- **KOBAYASHI, Y. ; FEARON, W. F.** Invasive coronary microcirculation assessment - Current status of Index of Microcirculatory Resistance. *Circulation Journal, Official Journal of the Japanese Circulation Society,* 2014, vol. 78 (5), 1021-1028